# EUROPÄISCHE PATENTANMELDUNG

(11) **EP 2 159 387 A1**
(43) Veröffentlichungstag der Anmeldung: **03.03.2010**
(21) Anmeldenummer: 08105198.9
(22) Anmeldetag: 01.09.2008
(51) Int. Cl.: F01M 11/10, G01N 11/16, G01N 33/28

(54) **Verfahren und Vorrichtung für einen mit aus Pflanzenöl hergestelltem Kraftstoff betriebenen Verbrennungsmotor**

(71) Anmelder: Pflanzenöltechnik Nord GmbH, 25524 Itzehoe (DE)
(72) Erfinder: Dr.-Ing. Bode, Berthold, 37412, Herzberg/Lonau (DE)
(74) Vertreter: Oberhardt, Knut

(57) **Zusammenfassung**

Die Erfindung geht aus von einem Verfahren für einen mit aus Pflanzenöl hergestelltem Kraftstoff betriebenen Verbrennungsmotor (1) zum Ermitteln des Zeitpunkts, zu dem das Schmieröl ausgetauscht werden muss. Erfindungsgemäß wird in einem Kalibrierungsschritt die Viskosität des frischen Schmieröls bestimmt und abgespeichert. Weiterhin werden nach der Durchführung des Kalibrierungsschritts kontinuierlich Viskositätsmessungen des Schmieröls durchgeführt und die ermittelten Werte mit dem abgespeicherten Wert verglichen. Bei einer ersten vorbestimmten Viskositätsänderung des Schmieröls wird auf einen fälligen Wechsel des Schmieröls geschlossen.

## Beschreibung

Die Erfindung betrifft ein Verfahren bzw. eine Vorrichtung für einen mit aus Pflanzenöl hergestelltem Kraftstoff betriebenen Verbrennungsmotor nach dem Oberbegriff von Anspruch 1, bzw. von Anspruch 15.

Im Zuge der Verteuerung von Erdöl und des daraus hergestellten Dieselkraftstoffs wurden Umbausysteme bekannt, die den Betrieb einer herkömmlichen dieselmotorischen Brennkraftmaschine mit reinem Pflanzenöl oder einem daraus hergestellten Kraftstoff ermöglichen. Da Pflanzenöl eine andere Verbrennungscharakteristik, wie Dieselkraftstoff zeigt, muss der Motor, insbesondere bezüglich des Einspritzverhaltens, an den neuen Kraftstoff angepasst werden. Trotz aller Bemühungen können während des Betriebs der Brennkraftmaschine jedoch immer wieder Betriebszustände vorkommen, bei denen das Pflanzenöl nicht vollständig verbrannt wird. Ohne dass bisher die genauen Umstände geklärt werden konnten, wurde nachgewiesen, dass auf diese Weise unverbranntes Pflanzenöl in das Schmieröl des Motors gelangen kann.

Pflanzenöl genügt jedoch nicht den Anforderungen, die moderne Brennkraftmaschinen an die Qualität des Schmieröls stellen. Auch eine Mischung aus einem modernen Schmieröl und Pflanzenöl verschlechtert, mit steigendem Anteil an Pflanzenöl, seine Schmierfähigkeit immer mehr. Es muss folglich verhindert werden, dass der Anteil an Pflanzenöl im Schmieröl eine bestimmte Grenze übersteigt.

Es wurde aber auch beobachtet, dass bereits kleine Anteile von Pflanzenöl im Schmieröl zur Polymerisation neigen. Die Bedingungen, die vorliegen müssen, damit es zur Polymerisation kommt, konnten noch nicht abschließend geklärt werden. Es wird jedoch angenommen, dass eine große Anzahl von Druck- und Temperaturwechseln für die Polymerisation verantwortlich ist. Bei dieser Polymerisation des Pflanzenöls entsteht eine sehr zähe Flüssigkeit, die ebenfalls nicht mehr die notwendige Schmierfähigkeit besitzt.

Bei mit Pflanzenöl oder einem daraus hergestellten Kraftstoff betriebenen Motoren hängt der Zeitpunkt, zu dem das Schmieröl gewechselt werden sollte, folglich weit stärker von den durchlaufenen Betriebszuständen und der Betriebsart des Motors ab als bei mit Dieselkrafstoff betriebenen Motoren. Das Festlegen eines festen Ölwechsel-Intervalls wäre deshalb sehr unwirtschaftlich, da aus Sicherheitsgründen relativ kurze Ölwechsel-Intervalle festgelegt werden müssten.

Der Erfindung liegt die Aufgabe zugrunde, ein Verfahren bzw. eine Vorrichtung für einen mit Pflanzenöl oder einem daraus hergestellten Kraftstoff betriebenen Motor zu entwickeln, womit der richtige Zeitpunkt für den Wechsel des Schmieröls sicher bestimmt werden kann. Damit soll verhindert werden, dass der, durch den Eintrag von Pflanzenöl verbundene Verlust der Schmierfähigkeit des Schmieröls zu groß wird und es zu einer Schädigung des Motors kommt.

Gelöst wird die Aufgabe gemäß der Erfindung durch ein Verfahren mit den Merkmalen von Anspruch 1 bzw. eine Vorrichtung mit den Merkmalen von Anspruch 15. Es hat sich herausgestellt, dass der Eintrag von Pflanzenöl in das Schmieröl am besten über die Viskosität des Schmieröls festgestellt werden kann. Allerdings ist eine absolute Bestimmung der Viskosität im praktischen Einsatz außerhalb des Labors nur schwer möglich. Es wird daher die Viskositätsänderung in Relation zur Viskosität des frisch eingefüllten Schmieröls nach einem Ölwechsel bestimmt. Zu diesem Zweck wird zuerst in einem Kalibrierungsschritt die Viskosität des frischen Schmieröls gemessen und abgespeichert.

Nach der Durchführung des Kalibrierungsschritts werden kontinuierlich Viskositätsmessungen des Schmieröls durchgeführt und die ermittelten Werte mit dem abgespeicherten Wert verglichen, so dass eine Maßzahl erhalten wird, die der Viskositätsänderung des Schmieröls entspricht. Bei einer vorbestimmten Viskositätsänderung gegenüber dem abgespeicherten Wert, wird auf einen fälligen Wechsel des Schmieröls geschlossen.

Bei der Bestimmung der Viskositätsänderung wird keine Rücksicht darauf genommen, ob die Maßzahl für die Viskositätsänderung ein negatives oder ein positives Vorzeichen besitzt. Auf diese Weise kann sowohl die Verflüssigung durch einen zu hohen Eintrag an Pflanzenöl, als auch eine Verdickung durch die Polymerisation des Pflanzenöls erfasst werden. Durch diese Maßnahme lässt sich erstmals, bei mit Pflanzenöl betreibbaren Motoren, der richtige Zeitpunkt zum Wechsel des Schmieröls mit großer Sicherheit bestimmen. Es lassen sich damit Motorschäden vermeiden, ohne dass gleichzeitig die Ölwechselintervalle unverhältnissmäßig verkürzt werden müssten. Dies führt zu einer Optimierung bei der Bestimmung der Ölwechselintervalle und damit zu einer gesteigerten Wirtschaftlichkeit beim Betrieb solcher Verbrennungsmotoren.

Die vorbestimmte Viskositätsänderung des Schmieröls, bei der auf einen fälligen Wechsel des Schmieröls geschlossen wird, liegt vorteilhaft zwischen 25 % und 35 %. Dieser Bereich ist auf die heute üblichen Schmieröle bezogen. Beim zukünftigen Einsatz weiterentwickelter Schmieröle oder bei einer Verbesserung der Motortechnik kann sich dieser Bereich jedoch verschieben. Idealerweise wird heute bei einer Viskositätsänderung von etwa 30 % auf die Fälligkeit des Ölwechsels geschlossen. Bei der Viskositätsänderung von 30 % ergibt sich ein Optimum zwischen der Vermeidung eines eventuellen Motorschadens und eines zu früh vorgenommenen Ölwechsels.

Vorteilhaft wird bei Erreichen der vorbestimmten Viskositätsänderung ein akustisches und/oder visuelles Alarmsignal ausgelöst. Bei einem akustischen Alarmsignal muss die Möglichkeit gegeben sein, dies abzustellen. Da ein Ölwechsel meist nie in der gegenwärtigen Situation sofort durchgeführt werden kann und üblicherweise der nächste Werkstattbesuch abgewartet werden muss, würde ein ständiges akustisches Alarmsignal zu einer Störung und starken Belästigung des Fahrers führen. Ist eine Möglichkeit zum Abschalten des akustischen Alarmsignals vorgesehen, kann jedoch der fällige Ölwechsel leicht in Vergessenheit geraten. Ist nur ein visuelles Alarmsignal vorgesehen, bedingt dieses zwar keine Störung des Fahrers, es kann aber leicht übersehen werden und somit unbemerkt bleiben.

Im Idealfall ist daher sowohl ein akustisches Alarmsignal in Verbindung mit einem visuellen Alarmsignal vorgesehen, wobei das akustische Alarmsignal vom Fahrer abgestellt werden kann. Auf diese Weise erhält der Fahrer einen Hinweis auf den fälligen Ölwechsel, der in jedem Fall bemerkt wird, wobei nach dem Abschalten des akustischen Alarmsignals das visuelle Alarmsignal eine Erinnerungsfunktion übernimmt.

Besonders vorteilhaft wird bei Erreichen einer zweiten vorbestimmten Viskositätsänderung ein akustisches oder visuelles Warnsignal generiert. Diese zweite vorbestimmte Viskositätsänderung ist kleiner als die erste vorbestimmte Viskositätsänderung angesetzt. Das Warnsignal dient der rechtzeitigen Information des Fahrers, dass in absehbarer Zeit ein Ölwechsel fällig wird. Der Fahrer kann sich so darauf einstellen und den nächsten Serviceaufenthalt in der Werkstatt bereits einplanen.

Die zweite vorbestimmte Viskositätsänderung liegt vorteilhaft zwischen 10 % und 20 %. Besonders vorteilhaft wird das Warnsignal aber bei einer Viskositätsänderung von 15 % generiert.

Die Viskosität von Schmieröl, und auch von Pflanzenöl ist stark temperaturabhängig. Mit steigender Temperatur wird Schmieröl immer dünnflüssiger, während bei niedrigen Temperaturen eher eine zähflüssige Beschaffenheit vorliegt. Um diesen Umstand bei der Ermittlung der Viskositätsänderung berücksichtigen zu können, wird zusammen mit der Viskosität immer die Temperatur des Schmieröls bestimmt.

Bei einem besonders einfachen Ausführungsbeispiel des erfindungsgemäßen Verfahrens erfolgt jede Viskositätsmessung bei gleicher Temperatur des Schmieröls. So kann beispielsweise eine Schmieröltemperatur von 55°C festgelegt werden, bei der die Viskositätsmessung stattfindet. Diese Temperatur wird regelmäßig nach dem Starten des Motors in der Phase durchschritten, in der die Brennkraftmaschine noch nicht ihre endgültige Betriebstemperatur erreicht hat. Ebenso durchschreitet das Schmieröl diesen Temperaturbereich erneut nach dem Abstellen der Brennkraftmaschine. In beiden Zuständen können Viskositätsmessungen bei der gleichen voreingestellten Temperatur durchgeführt werden. Sinnvollerweise wird jedoch während des Abkühlvorgangs gemessen, da hier der maßgebliche Temperaturbereich langsamer durchschritten wird und somit eine genauere Viskositätsmessung möglich ist. Bei diesem Ausführungsbeispiel wird auch die Viskosität des frisch eingefüllten Schmieröls, nach dem Ölwechsel, bei der gleichen Temperatur ermittelt.

Bei einem anderen Ausführungsbeispiel erfolgt die Viskositätsmessung bei der jeweils vorherrschenden Temperatur des Schmieröls, ohne dass eine bestimmte Temperatur festgesetzt worden wäre. Dieses Verfahren ist aufwändiger, jedoch können häufiger Viskositätsmessungen vorgenommen werden. Dieses Verfahren eignet sich auch dann besonders, wenn vorher nicht bekannt ist, welche Temperaturbereiche von dem Schmieröl durchlaufen werden, oder wenn der Verbrennungsmotor nur sehr selten abgeschaltet wird.

Für dieses Verfahren muss in einer Steuerung eine Viskositäts-Temperatur-Beziehung des Schmieröls hinterlegt werden. Die Hinterlegung findet üblicherweise in einer sogenannten LUT (Look-Up-Table) statt. Das Einlesen der Werte kann auf verschiedene Arten vorgenommen werden. Im einfachsten Fall wird ein bestimmtes handelsübliches Schmieröl für den Einsatz vorgeschrieben. Bei diesem bekannten Schmieröl ist die Viskositäts-Temperatur-Beziehung ebenfalls bekannt. Diese bekannten Werte können hinterlegt werden, wobei davon ausgegangen werden kann, dass bei der Verwendung des vorgeschriebenen Schmieröls dann keine gesonderten Messungen zur Ermittlung der Viskositäts-Temperatur-Beziehung mehr durchgeführt werden müssen.

Wird dagegen kein bestimmtes Schmieröl für die Verwendung vorgeschrieben, so kann auch keine feste Viskositäts-Temperatur-Beziehung hinterlegt werden. In diesem Fall wird die Viskositäts-Temperatur-Beziehung nach der Gleichung von Ubbelohde-Walter ermittelt. Bei der Ermittlung der Viskositäts-Temperatur-Beziehung nach dieser Gleichung werden grundsätzlich zwei, bei unterschiedlichen Temperaturen gemessene Viskositätswerte benötigt.

Nach einem Ölwechsel werden bei diesem Verfahren folglich zwei Viskositätsmessungen bei unterschiedlichen Temperaturen durchgeführt, wobei die Genauigkeit der ermittelten Viskositäts-Temperatur-Beziehung steigt, wenn die beiden Temperaturen möglichst weit auseinander liegen. Die so ermittelte Viskositäts-Temperatur-Beziehung des Schmieröls wird wiederum in der LUT abgespeichert.

Ist die Klassifizierung des verwendeten Schmieröls bekannt, ist eine Viskositätsmessung bei einer Temperatur ausreichend. Die Viskosität bei einer weiteren Temperatur kann dann auf Basis der Klassifizierungsangabe des Schmieröls interpoliert werden.

Besonders vorteilhaft wird jeweils auch die Permittivitätszahl oder relative Permittivität des Schmieröls bestimmt. Die relative Permittivität des Schmieröls verändert sich durch den Eintrag von Pflanzenöl kaum. Eine erhebliche Änderung der relativen Permittivität findet aber dann statt, wenn beispielsweise Wasser in das Schmieröl gelangt.

Bei einem Wassereintrag in das Schmieröl sind jedoch andere Aspekte zu beachten und es müssen andere Warn- und Alarmsignale generiert werden. Auf einem zu hohen Anteil an des aus Pflanzenöl hergestellten Kraftstoffs im Schmieröl und die Fälligkeit des damit verbundenen Ölwechsels wird deshalb nur dann gefolgert, wenn bei einer vorbestimmten Viskositätsänderung keine zusätzliche, erhebliche Änderung der relativen Permittivität festgestellt wird.

Die erfindungsgemäße Vorrichtung weist einen Sensor zur Messung der Viskosität des Schmieröls und eine damit verbundene Steuerung auf. Die Steuerung kann als Platine aufgebaut sein, die sich in die Motorsteuerung integrieren lässt. Ist die Steuerung aber in einem eigenen Gehäuse untergebracht eignet sich die Einheit aus Steuerung und Sensor auch hervorragend zum nachträglichen Einbau.

Die Steuerung weist vorteilhaft verschiedenfarbige LEDs zur Anzeige des Anteils an Kraftstoff im Schmieröl des Verbrennungsmotors auf. So lässt sich beispielsweise eine grüne LED vorsehen, um dem Fahrer zu signalisieren, dass sich das Schmieröl in einem guten Zustand befindet. Eine rote LED wird üblicherweise als Alarmsignal interpretiert und wird auch hier eingesetzt, um dem Fahrer anzuzeigen, dass der Wechsel des Schmieröls dringend geboten ist. Vorteilhaft kann zusätzlich noch eine gelbe LED vorgesehen sein, die den Fahrer dazu veranlassen soll, den nächsten Ölwechsel bereits einzuplanen. Leuchtet diese gelbe LED auf, ist zwar noch nicht mit einer Schädigung des Verbrennungsmotors zu rechnen, es zeigt jedoch an, dass sich der Zustand des Schmieröls verschlechtert hat und bald ein Zustand erreicht wird, der den Wechsel des Schmieröls dringend erforderlich macht.

Vorteilhaft weist der Sensor einen piezoelektrischen Torsionsschwingerkristall auf. Diese Torsionsschwingerkristalle reinigen sich selbst und sind deshalb hervorragend für den Einsatz in Schmieröl geeignet. Selbst bei verschmutztem Schmieröl liefern sie noch sehr gute Messergebnisse.

In einem vorteilhaften Ausführungsbeispiel weist der Sensor zur Messung der Viskosität des Schmieröls zusätzlich einen Temperaturdetektor und eine Einrichtung zur Bestimmung der relativen Permittivität auf. Der Sensor kann so sehr kompakt aufgebaut werden und liefert alle Messwerte, die für die Bestimmung des richtigen Zeitpunkts für den Ölwechsel benötigt werden. Gleichzeitig liefert dieser Sensor aber auch noch den Messwert, mit dem das Eindringen von Kühlwasser in das Schmieröl des Motors festgestellt werden kann.

Bei einer besonders kompakten Version kann der Torsionsschwingerkristall nicht nur zur Viskositätsmessung, sondern ebenso zur Messung der relativen Permittivität verwendet werden. Genauere Messergebnisse lassen sich allerdings erreichen, wenn an der Einrichtung zur Bestimmung der relativen Permittivität zusätzliche Elektrodenflächen realisiert werden können.

Der Sensor ist besonders vorteilhaft in der Ölwanne des Verbrennungsmotors angeordnet. Diese Position eignet sich auch hervorragend für einen nachträglichen Einbau. Der Sensor befindet sich hier immer innerhalb des Schmierölsumpfes, so dass Messungen jederzeit durchgeführt werden können.

Erfindungsgemäß ist ein Bedienelement zum Auslösen eines Messvorgangs nach einem Wechsel des Schmieröls vorgesehen. Dieses Bedienelement kann an der Steuerung angebracht sein. Das Bedienelement kann beispielsweise mit entsprechenden Leuchtmitteln versehen sein, so dass die Gefahr, den Kalibriervorgang nach einem Ölwechsel zu vergessen, nur sehr gering ist.

Die Vorrichtung besteht grundsätzlich aus zwei Komponenten: einem Ölsensor und einer kleinen Platine mit der entsprechenden Ansteuer- u. Auswerteelektronik, die vorzugsweise im Steuergerät eines LKW integriert ist oder im Falle der Nachrüstung in ein kleines Gehäuse im Cockpit des LKW eingesetzt ist. Der Einbau des Ölsensors erfolgt bevorzugt direkt in der Ölwanne; dieser Einbau ist denkbar einfach, da der Ölsensor unabhängig von der Strömungsform des Schmieröls (ruhend, laminar oder turbulent) arbeitet.

Die erfindungsgemäße Vorrichtung erfordert keinerlei Wartung, arbeitet ohne mechanische Verschleißteile und kann aufgrund seiner kleinen modularen Bauweise praktisch jedem Aggregat angepaßt werden kann.

Ein wichtiger Aspekt der Vorrichtung ist die hinreichend genaue Bestimmung des aktuellen Anteils an Pflanzenöl im Motoröl. Dieses Problem wird anhand der aktuell gemessenen Motorölviskosität gelöst, die sich bei näherer Betrachtung als eine Mischviskosität darstellt. Es überlagern sich hierbei zwei Viskositäten, - die Viskosität des reinen Motoröles einerseits und die Viskosität des Pflanzenöles andererseits. Mit Hilfe einer geeigneten, in der Steuerung hinterlegten Mischungsregel kann nun auf den prozentualen Anteil des Pflanzenöles (mittlere Viskosität) im Motoröl (hohe Viskosität) geschlossen werden, da die Viskosität des reinen Motoröls der Steuerung durch den erfolgten Kalibriervorgang nach dem Ölwechsel bekannt ist.

Da nun aber insbesondere die Stoffeigenschaft "Viskosität" ganz entscheidend auch von der aktuellen Temperatur abhängt ist die gleichzeitige Erfassung dieser Betriebsgröße von grundlegender Bedeutung. Sollen Viskositäten oder auch nur Viskositätsänderungen miteinander verglichen werden, so ist hierfür eine gemeinsame Bezugstemperatur unerläßlich.

Im Laborbereich werden derartige Stoffuntersuchungen stets bei definierter Isothermentemperatur durchgeführt; hierfür werden in der Regel bestimmte Fixtemperaturen vorgegeben und vor der Durchführung der eigentlichen Messung wird hinreichend lange auf das sich einstellende Temperaturgleichgewicht gewartet. Derartig ideale Bedingungen liegen in der Regel bei einem stationären oder mobilen Verbrennungsmotor nicht vor.

Grundsätzlich gibt es hier nun zwei Möglichkeiten, die jeweilige Viskositätsänderung zu bestimmen:
- die Messungen zur Bestimmung der Viskositätsänderung des Schmieröls und des Pflanzenölanteiles erfolgen stets bei einer in der Steuerung frei programmierbaren Referenztemperatur,
- sowohl die auf die Messung nach dem Ölwechsel bezogene Viskositätsänderung als auch die daraus abgeleitete Angabe des Pflanzenölanteils wird bei beliebiger Temperatur ermittelt.

Die Datengenerierung und -interpretation bei definierter Referenztemperatur hat den Vorteil, dass sie deutlich übersichtlicher ist und jegliche Extrapolationsfehler ausgeschlossen sind. Die so gewonnen Daten können ohne weiteren Aufwand direkt miteinander verglichen werden, da sie allesamt bei der gleichen Temperatur ermittelt worden sind. Dies setzt allerdings voraus, dass sich stets auch die definierte Referenztemperatur einstellt, was bei einem Motor, der mit unterschiedlichsten Lastprofilen betrieben wird, zumindest während des normalen Betriebs kaum gegeben ist. Eine Messung bei definierter Referenztemperatur ist aber insbesondere nach dem Ausschalten des Motors möglich, wenn man sich die Abkühlung des Motoröls über die Umgebungstemperatur zu Nutze machen kann.

Im Falle der Datengenerierung bei beliebiger Temperatur ist die Kenntnis der Temperaturabhängigkeit der Viskosität unabdingbar. Aber auch hier ist es wieder sinnvoll, die ermittelten Daten auf eine Vergleichstemperatur zu beziehen. Als geeigneter Lösungsansatz zur Beschreibung der Viskosität als Funktion der Temperatur hat sich die Gleichung von Ubbelohde-Walter erwiesen. Hier stellt sich der Viskositätsverlauf über der Temperatur durch die doppelt-logarithmische Skalierung als Gerade dar. Ist die Steigung dieser Geraden bekannt (die sog. Richtungscharakteristik m), lässt sich - ausgehend von der gemessenen Viskosität bei der aktuellen Temperatur - problemlos die gesuchte Viskosität bei der Vergleichstemperatur berechnen.

Auch für die Ermittlung der gesuchten Richtungscharakteristik m gibt es wiederum zwei Möglichkeiten:
- die Vorrichtung ermittelt die Viskosität des Motoröles bei mindestens zwei unterschiedlichen Temperaturen und berechnet daraus die gesuchte Richtungscharakteristik m,
- da Schmieröle einer Klassifizierung unterliegen, lässt sich bei Kenntnis der Viskosität bei nur einer Temperatur hinreichend genau auf die Richtungscharakteristik m schließen.

Weitere Einzelheiten und Vorteile der Erfindung ergeben sich aus den Unteransprüchen im Zusammenhang mit der Beschreibung eines Ausführungsbeispiels, das anhand der Zeichnung eingehend erläutert wird.

Es zeigt:
- Fig. 1: eine schematische Ansicht eines Verbrennungsmotors und der erfindungsgemäßen Vorrichtung,
- Fig. 2: ein Ausführungsbeispiel für einen Ölsensor und
- Fig. 3: ein Ablaufschema für das erfindungsgemäße Verfahren.

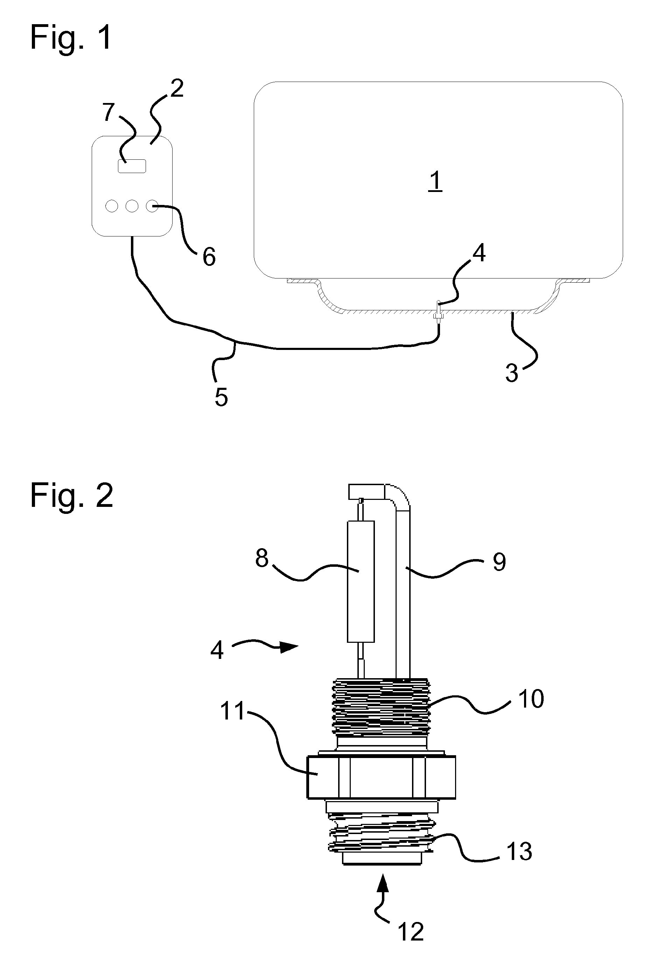

Der Motor 1 ist in dem in Fig. 1 gezeigten Ausführungsbeispiel als dieselmotorisch betriebene Brennkraftmaschine ausgeführt. Diese Brennkraftmaschine wurde speziell für den Betrieb mit reinem Pflanzenöl umgerüstet und an die unterschiedliche Verbrennungscharakteristik angepasst.

Die Ölwanne 3 unterhalb des Motorblocks ist geschnitten dargestellt, so dass der darin angebrachte Ölsensor 4 sichtbar ist. Der Ölsensor 4 ist über das Kabel 5 mit einer Steuerung 2 verbunden. Das Kabel 5 beinhaltet Datenleitungen für den Datentransfer zwischen Ölsensor 4 und Steuerung 2.

Die Steuerung 2 ist so angebracht, dass sie von einer Bedienperson gut eingesehen werden kann. Wird die Vorrichtung beispielsweise in einem LKW verwendet, ist die Steuerung 2 vorteilhaft in der Fahrerkabine des LKWs angeordnet. Wird der Motor 1 dagegen im stationären Betrieb als Antrieb für einen Stromerzeuger oder ein Blockheizkraftwerk verwendet, ist es sinnvoll, die Steuerung 2 entweder in der Nähe des Motors 1 oder eines Bedienfeldes, von dem aus der Motor 1 kontrolliert wird, unterzubringen.

Die Steuerung weist drei LEDs 6 zur Anzeige verschiedener Zustände des Schmieröls auf. Um diese Zustände klar unterscheiden zu können, ist es beispielsweise möglich, drei gleichfarbige LEDs zu verwenden, die aber von unterschiedlich gefärbten Feldern umrahmt sind. So könnte beispielsweise die linke LED von einem grünen Feld, die mittlere LED von einem gelben Feld und die rechte LED von einem roten Feld umrahmt sein. Einfacher ist es jedoch unterschiedlich gefärbte LEDs zu verwenden. Durch die Farbe Grün wird signalisiert, dass sich das Schmieröl in einem guten Zustand befindet und keinerlei Gefahr für eine Beschädigung des Motors wegen schlechter Schmierölqualität besteht. Die Farbe gelb wird allgemein mit dem Begriff "Achtung" in Verbindung gebracht. Auch hier bedeutet Gelb, dass sich der Zustand des Schmieröls verschlechtert hat, dass aber immer noch keine Gefahr für eine Schädigung des Motors besteht. Allerdings sollte der Fahrer bei diesem Schmierölzustand bereits den nächsten Ölwechsel einplanen. Beim Umspringen der Anzeige auf die Farbe Rot wird signalisiert, dass der der nächste Ölwechsel schnell zu erfolgen hat. Bei diesem Schmierölzustand sollte dem Motor 1 keine Dauerleistung im Hochlastbereich mehr abverlangt werden. Für diesen Fall könnte eine Schädigung des Motors 1 nicht mehr ausgeschlossen werden.

Weiterhin ist an der Steuerung 2 der Betätigungsknopf 7 vorgesehen. Dieser Betätigungsknopf 7 dient dazu, nach einem Ölwechsel einen Kalibriervorgang auszulösen. Wird der Betätigungsknopf 7 gedrückt, leitet die Steuerung 2 einen Messvorgang ein und speichert die ermittelten Werte, für den Vergleich mit späteren Werten, ab.

Um sicher zu stellen, dass der Kalibriervorgang nach einem Ölwechsel nicht vergessen wird, sind unterschiedliche Möglichkeiten vorstellbar. Bei einer sehr einfachen Ausführungsform ist der Betätigungsknopf 7 mit einem Leuchtmittel versehen, welches jeweils nach dem Start des Motors 1 zu blinken beginnt. Wurde kein Ölwechsel durchgeführt, lässt sich das Leuchtmittel durch einen kurzen Druck auf den Betätigungsknopf 7 abstellen. Wurde jedoch ein Ölwechsel durchgeführt, kann der Kalibriervorgang durch ein längeres Drücken des Betätigungsknopfes 7 ausgelöst werden. Auch in diesem Fall wird das Leuchtmittel wieder abgeschaltet.

Ebenso ist es möglich einen Betätigungsknopf zum Auslösen eines Kalibriervorgangs so anzubringen, dass er nur mit einem speziellen Werkzeug betätigt werden kann. Das Durchführen der Neukalibrierung ist in diesem Fall nicht die Aufgabe des Fahrers, sondern des Service-Personals, welches den Ölwechsel durchgeführt hat.

Bei einem komfortableren Ausführungsbeispiel wird das Leuchtmittel nur eingeschaltet, wenn zwischen zwei Messungen eine sprunghafte Viskositätsänderung des Schmieröls auftritt. Dies ist regelmäßig bei einem Ölwechsel der Fall. Auch hier kann dann der Fahrer, durch einen längeren Druck auf den Betätigungsknopf 7, den Kalibriervorgang auslösen. Sollte kein Ölwechsel durchgeführt worden sein, ist das Blinken des Betätigungsknopfes 7 ein Hinweis an den Fahrer, für ein anderweitiges schwerwiegendes Motorproblem.

In Fig. 2 ist ein Ölsensor dargestellt, wie er in der Ölwanne 3 eingesetzt werden kann. Der Ölsensor 4 wird durch eine Bohrung im Bodenblech der Ölwanne 3 gesteckt und von der Innenseite mit einer hier nicht gezeigten Mutter auf dem Befestigungsgewinde 10 gesichert. Zur Herstellung einer festen Klemmverbindung ist der Schraubkopf 11 vorgesehen. Zwischen dem Schraubkopf 11 und dem Bodenblech der Ölwanne 3 wird vorteilhaft ein Dichtring eingesetzt.

Im Innenbereich der Ölwanne 3 weist der Ölsensor 4 einen Haltebügel 9 zum Halten des Torsionsquarzes 8 auf. Mit Hilfe dieses Torsionsquarzes 8 wird die Viskosität des Schmieröls bestimmt. Über hier nicht dargestellte Isolierungen zwischen einzelnen Teilen des Ölsensors 4 im Innenraum der Ölwanne 3 werden Kapazitäten gebildet, mit denen die relative Permittivität des Schmieröls bestimmt werden kann. Ebenso ist ein Temperatursensor vorgesehen, der in dieser Darstellung nicht sichtbar ist.

Im außerhalb der Ölwanne 3 liegenden Bereich des Ölsensors 4 befindet sich das Stecksystem 12 mit der Schraubverriegelung 13 zum Anschluss des Kabels 5. Hier werden über Steckverbindungen die Datenleitungen angekoppelt.

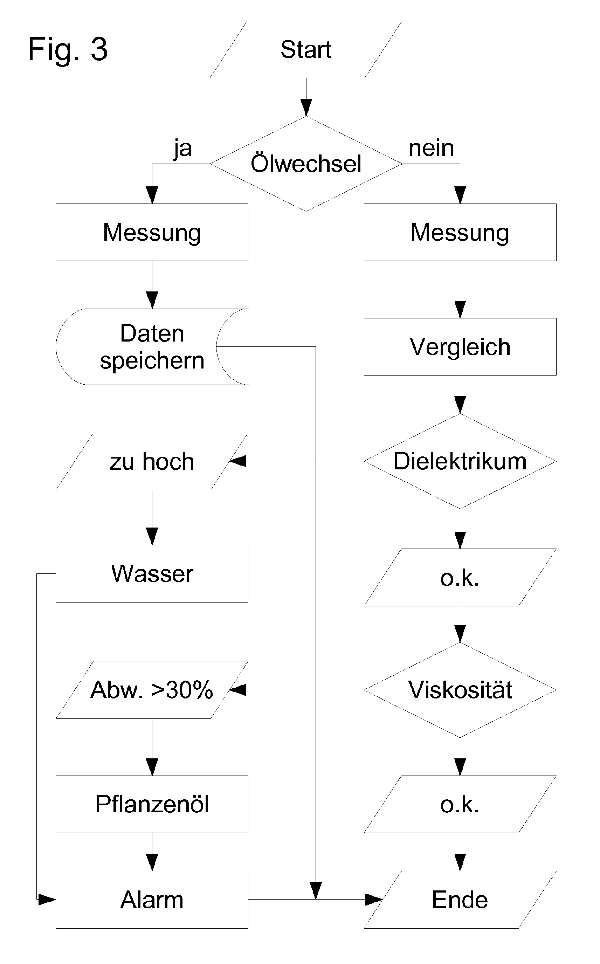

Im Folgenden soll ein Ausführungsbeispiel des erfindungsgemäßen Verfahrens anhand von Fig. 3 näher erläutert werden. Bei dem dargestellten Ablaufschema wurde davon ausgegangen, dass bei einem LKW jeweils nach dem Start, sobald das Schmieröl eine vorbestimmte Temperatur von beispielsweise 55°C erreicht hat, eine Messung durchgeführt wird. Ist beispielsweise ein Ölwechsel erfolgt und es befindet sich frisches Schmieröl in der Ölwanne 3, teilt dies der Fahrer dem System über ein Drücken des Betätigungsknopfes 7 mit. Sobald die Temperatur von 55°C erreicht ist, wird die stattgefundene Betätigung des Betätigungsknopfes 7 abgefragt. Wurde der Betätigungsknopf 7 gedrückt und ein Ölwechsel durchgeführt, wird ein Kalibrierungsvorgang ausgeführt mit den Schritten, die im linken Zweig des Ablaufdiagramms dargestellt sind.

Im ersten Schritt wird eine Messung der Viskosität, mit Hilfe des Torsionsquarzes 8, in dem Ölsensor 4 durchgeführt. In einem zweiten Schritt werden die Daten dieser Messung in der Steuerung 2 abgespeichert. In dem hier gezeigten Ausführungsbeispiel ist es ausreichend, den Wert für die Viskositätsmessung abzuspeichern. Bei einem Ausführungsbeispiel, bei dem bei unterschiedlichen Öltemperaturen gemessen wird, muss die Temperatur zusätzlich abgespeichert werden. Vorteilhaft ist es auch weiterhin den Messwert für die relative Permittivität mit abzuspeichern. Nach dem Abspeichern der Messdaten wird der Kalibriervorgang beendet.

Wird nach dem Start des Motors 1, bei Erreichen der Temperatur von 55°C in der Ölwanne 3 festgestellt, dass der Betätigungsknopf 7 nicht gedrückt worden ist und somit kein Ölwechsel stattgefunden hat, werden nacheinander die Schritte ausgeführt, die in dem rechten Ast des Ablaufschemas dargestellt sind. Auch hier findet im ersten Schritt eine Messung mit dem Ölsensor 4 statt. Es wird zumindest ein Wert für die Viskosität des Schmieröls und ein Wert für die relative Permittivität ermittelt.

Im zweiten Schritt werden die ermittelten Werte mit den Werten verglichen, die während des Kalibriervorgangs in der Steuerung 2 abgespeichert wurden. Zuerst werden die Werte für die relative Permittivität miteinander verglichen. Ist der aktuell gemessene Wert gegenüber dem abgespeicherten Wert sehr hoch, wird auf einen Wassereintrag in das Schmieröl geschlossen. Da sich die relative Permittivität von Schmieröl und Wasser sehr stark unterscheidet, muss dieser Wert nicht unbedingt mit dem Kalibrierwert verglichen werden. Es könnte hier unter Umständen auch eine absolute Messung vorgenommen werden. In diesem Fall würde bei Überschreitung eines bestimmten Absolutbetrags, auf den Wassereintrag rückgeschlossen werden. In jedem Fall wird hier sofort ein Alarm generiert, da die Gefahr einer Motorschädigung sehr groß ist. Der Messablauf wird danach beendet.

Ist der gemessene Wert für die relative Permittivität in Ordnung, wird im nächsten Schritt die ermittelte Viskosität mit dem in der Steuerung 2 abgespeicherten Wert verglichen. Beträgt die Abweichung gegenüber dem gespeicherten Wert mehr als 30 %, wird auf einen Pflanzenölgehalt des Schmieröls geschlossen, bei dem sich eine Schädigung des Motors 1 nicht mehr ausschließen lässt. In diesem Fall wird ebenfalls ein Alarm generiert und der Messablauf abgeschlossen. Bei dem in Fig. 1 dargestellten Ausführungsbeispiel der Steuerung 2, leuchtet in diesem Fall eine rote LED auf. Um auszuschließen, dass diese Anzeige übersehen wird, kann zusätzlich ein akustisches Alarmsignal generiert werden.

Bei der Ausgestaltung der Steuerung 2 mit drei LEDs, müsste in dem Ablaufschema noch ein weiterer, hier nicht aufgezeigter Vergleichsschritt eingebaut werden. In diesem Fall würde in einem zweiten Schritt, noch vor der Okay-Box festgestellt, ob die Abweichung mehr als 15 % beträgt. Trifft dies zu, würde ein Warnsignal generiert werden. In dem in Fig. 1 dargestellten Ausführungsbeispiel der Steuerung 2 würde eine gelbe LED aufleuchten.

Ist die ermittelte Abweichung der Viskosität geringer als 30 % bzw. als 15 %, ist der Zustand des Schmieröls in Ordnung und es leuchtet die grüne LED der Steuerung 2.

Das hier gezeigte Ablaufschema ist für eine sehr einfache Messung bei einem LKW oder auch PKW geeignet. Nach der Erfindung sind jedoch auch Abläufe möglich, die eine komfortablere Bedienung ermöglichen, wie auch Abläufe, die beispielsweise für Stationärmotoren, Anwendung finden können.

Das System ermöglicht eine kontinuierliche, rückwirkungs- und zerstörungsfreie Schmierölüberwachung von Motoren, die mit einem aus Pflanzenöl hergestellten Kraftstoff betrieben werden. Es ist dazu geeignet, den prozentualen Anteil von Pflanzenöl im Schmieröl des Motors zu bestimmen und liefert erstmalig einen entscheidenden Beitrag zur Schadensprävention, sowie wichtige Informationen im Hinblick auf eine Optimierung der Ölwechsel-Intervalle bei den mit Pflanzenöl betriebenen Motoren.

### Bezugszeichenliste:

- 1: Motor
- 2: Steuerung
- 3: Ölwanne
- 4: Ölsensor
- 5: Kabel
- 6: LEDs
- 7: Betätigungsknopf
- 8: Torsionsquarz
- 9: Haltebügel
- 10: Befestigungsgewinde
- 11: Schraubkopf
- 12: Stecksystem
- 13: Schraubverriegelung

## Patentansprüche

1. Verfahren für einen mit aus Pflanzenöl hergestelltem Kraftstoff betriebenen Verbrennungsmotor (1) zum Ermitteln des Zeitpunkts, zu dem das Schmieröl ausgetauscht werden muss, **dadurch gekennzeichnet, dass** in einem Kalibrierungsschritt die Viskosität des frischen Schmieröls bestimmt und abgespeichert wird, dass nach der Durchführung des Kalibrierungsschritts kontinuierlich Viskositätsmessungen des Schmieröls durchgeführt und die ermittelten Werte mit dem abgespeicherten Wert verglichen werden und dass bei einer ersten vorbestimmten Viskositätsänderung des Schmieröls auf einen fälligen Wechsel des Schmieröls geschlossen wird.

2. Verfahren nach Anspruch 1, **dadurch gekennzeichnet, dass** die erste vorbestimmte Viskositätsänderung des Schmieröls zwischen 25 und 35% liegt.

3. Verfahren nach Anspruch 1, **dadurch gekennzeichnet, dass** bei Erreichen der vorbestimmten Viskositätsänderung ein akustisches und/oder visuelles Alarmsignal ausgelöst wird.

4. Verfahren nach Anspruch 3, **dadurch gekennzeichnet, dass** bei Erreichen einer zweiten vorbestimmten Viskositätsänderung ein akustisches und/oder visuelles Warnsignal generiert wird.

5. Verfahren nach Anspruch 4, **dadurch gekennzeichnet, dass** die zweite vorbestimmte Viskositätsänderung zwischen 10 und 20% liegt.

6. Verfahren nach Anspruch 1, **dadurch gekennzeichnet, dass** zusammen mit der Viskosität die Temperatur des Schmieröls bestimmt wird.

7. Verfahren nach Anspruch 6, **dadurch gekennzeichnet, dass** jede Viskositätsmessung bei gleicher Temperatur des Schmieröls erfolgt.

8. Verfahren nach Anspruch 6, **dadurch gekennzeichnet, dass** die Viskositätsmessung bei der jeweils vorherrschenden Temperatur des Schmieröls erfolgt.

9. Verfahren nach Anspruch 8, **dadurch gekennzeichnet, dass** eine Viskosität-Temperatur-Beziehung des Schmieröls in einer Steuerung (2) hinterlegt wird.

10. Verfahren nach Anspruch 9, **dadurch gekennzeichnet, dass** die Viskositäts-Temperatur-Beziehung nach Ubbelohde-Walter ermittelt wird.

11. Verfahren nach Anspruch 10, **dadurch gekennzeichnet, dass** die Viskosität-Temperatur-Beziehung durch zwei Viskositätsmessungen bei unterschiedlichen Temperaturen ermittelt wird.

12. Verfahren nach Anspruch 10, **dadurch gekennzeichnet, dass** die Viskosität-Temperatur-Beziehung des Schmieröls durch eine Viskositätsmessung und eine Temperaturmessung, sowie die Klassifizierungsangabe des Schmieröls ermittelt wird.

13. Verfahren nach Anspruch 1, **dadurch gekennzeichnet, dass** die relative Permittivität des Schmieröls bestimmt wird.

14. Verfahren nach Anspruch 13, **dadurch gekennzeichnet, dass** ein zu hoher Anteil an des aus Pflanzenöl hergestellten Kraftstoffs im Schmieröl nur dann gefolgert wird, wenn bei einer vorbestimmten Viskositätsänderung keine zusätzliche erhebliche Änderung der relativen Permittivität festgestellt wird.

15. Vorrichtung für einen mit aus Pflanzenöl hergestelltem Kraftstoff betriebenen Verbrennungsmotor (1) zum Ermitteln des Zeitpunkts, zu dem das Schmieröl ausgetauscht werden muss, **dadurch gekennzeichnet, dass** die Vorrichtung einen Sensor (4) zur Messung der Viskosität des Schmieröls aufweist und dass eine Steuerung (2) vorgesehen ist, die so aufgebaut ist, dass die Viskosität des frischen Schmieröls gemessen und abgespeichert wird, dass kontinuierlich Viskositätsmessungen des Schmieröls durchgeführt und die ermittelten Werte mit dem abgespeicherten Wert verglichen werden und dass bei einer ersten vorbestimmten Viskositätsänderung des Schmieröls auf einen fälligen Wechsel des Schmieröls geschlossen wird.

16. Vorrichtung nach Anspruch 15, **dadurch gekennzeichnet, dass** die Steuerung (2) verschiedenfarbige LEDs (6) zur Anzeige des Anteils des Kraftstoffs im Schmieröl des Verbrennungsmotors (1) aufweist.

17. Vorrichtung nach Anspruch 15, **dadurch gekennzeichnet, dass** der Sensor (4) einen piezo-elektrischen Torsionsschwingerkristall (8) aufweist.

18. Vorrichtung nach Anspruch 15, **dadurch gekennzeichnet, dass** der Sensor (4) einen Temperatur-Detektor und eine Einrichtung zur Bestimmung der relativen Permittivität aufweist.

19. Vorrichtung nach Anspruch 15, **dadurch gekennzeichnet, dass** der Sensor (4) in der Ölwanne (3) des Verbrennungsmotors (1) angeordnet ist.

20. Vorrichtung nach Anspruch 15, **dadurch gekennzeichnet, dass** ein Bedienelement (7) zum Auslösen eines Messvorgangs nach einem Wechsel des Schmieröls vorgesehen ist.

## Geänderte Patentansprüche

### Geänderte Patentansprüche gemäss Regel 137(2) EPÜ.

**1.** Verfahren für einen mit aus Pflanzenöl hergestelltem Kraftstoff betriebenen Verbrennungsmotor (1) zum Ermitteln des Zeitpunkts, zu dem das Schmieröl ausgetauscht werden muss, **dadurch gekennzeichnet, dass** in einem Kalibrierungsschritt die Viskosität des frischen Schmieröls mit einem in der Ölwanne des Verbrennungsmotors (1) angeordneten Sensor (4) bestimmt und abgespeichert wird, dass nach der Durchführung des Kalibrierungsschritts kontinuierlich Viskositätsmessungen des Schmieröls mit dem Sensor (4) durchgeführt und die ermittelten Werte mit dem abgespeicherten Wert verglichen werden und dass bei einer ersten vorbestimmten negativen oder positiven Viskositätsänderung des Schmieröls auf einen fälligen Wechsel des Schmieröls geschlossen wird.

**2.** Verfahren nach Anspruch 1, **dadurch gekennzeichnet, dass** die erste vorbestimmte Viskositätsänderung des Schmieröls zwischen 25 und 35% liegt.

**3.** Verfahren nach Anspruch 1, **dadurch gekennzeichnet, dass** bei Erreichen der vorbestimmten Viskositätsänderung ein akustisches und/oder visuelles Alarmsignal ausgelöst wird.

**4.** Verfahren nach Anspruch 3, **dadurch gekennzeichnet, dass** bei Erreichen einer zweiten vorbestimmten Viskositätsänderung ein akustisches und/oder visuelles Warnsignal generiert wird.

**5.** Verfahren nach Anspruch 4, **dadurch gekennzeichnet, dass** die zweite vorbestimmte Viskositätsänderung zwischen 10 und 20% liegt.

**6.** Verfahren nach Anspruch 1, **dadurch gekennzeichnet, dass** zusammen mit der Viskosität die Temperatur des Schmieröls bestimmt wird.

**7.** Verfahren nach Anspruch 6, **dadurch gekennzeichnet, dass** jede Viskositätsmessung bei gleicher Temperatur des Schmieröls erfolgt.

**8.** Verfahren nach Anspruch 6, **dadurch gekennzeichnet, dass** die Viskositätsmessung bei der jeweils vorherrschenden Temperatur des Schmieröls erfolgt.

**9.** Verfahren nach Anspruch 8, **dadurch gekennzeichnet, dass** eine Viskosität-Temperatur-Beziehung des Schmieröls in einer Steuerung (2) hinterlegt wird.

**10.** Verfahren nach Anspruch 9, **dadurch gekennzeichnet, dass** die Viskositäts-Temperatur-Beziehung nach Ubbelohde-Walter ermittelt wird.

**11.** Verfahren nach Anspruch 10, **dadurch gekennzeichnet, dass** die Viskosität-Temperatur-Beziehung durch zwei Viskositätsmessungen bei unterschiedlichen Temperaturen ermittelt wird.

**12.** Verfahren nach Anspruch 10, **dadurch gekennzeichnet, dass** die Viskosität-Temperatur-Beziehung des Schmieröls durch eine Viskositätsmessung und eine Temperaturmessung, sowie die Klassifizierungsangabe des Schmieröls ermittelt wird.

**13.** Verfahren nach Anspruch 1, **dadurch gekennzeichnet, dass** die relative Permittivität des Schmieröls bestimmt wird.

**14.** Verfahren nach Anspruch 13, **dadurch gekennzeichnet, dass** ein zu hoher Anteil an des aus Pflanzenöl hergestellten Kraftstoffs im Schmieröl nur dann gefolgert wird, wenn bei einer vorbestimmten Viskositätsänderung keine zusätzliche erhebliche Änderung der relativen Permittivität festgestellt wird.

**15.** Vorrichtung für einen mit aus Pflanzenöl hergestelltem Kraftstoff betriebenen Verbrennungsmotor (1) zum Ermitteln des Zeitpunkts, zu dem das Schmieröl ausgetauscht werden muss, **dadurch gekennzeichnet, dass** die Vorrichtung einen Sensor (4) zur Messung der Viskosität des Schmieröls aufweist und dass eine Steuerung (2) vorgesehen ist, die so aufgebaut ist, dass die Viskosität des frischen Schmieröls gemessen und abgespeichert wird, dass kontinuierlich Viskositätsmessungen des Schmieröls durchgeführt und die ermittelten Werte mit dem abgespeicherten Wert verglichen werden und dass bei einer ersten vorbestimmten Viskositätsänderung des Schmieröls auf einen fälligen Wechsel des Schmieröls geschlossen wird.

**16.** Vorrichtung nach Anspruch 15, **dadurch gekennzeichnet, dass** die Steuerung (2) verschiedenfarbige LEDs (6) zur Anzeige des Anteils des Kraftstoffs im Schmieröl des Verbrennungsmotors (1) aufweist.

**17.** Vorrichtung nach Anspruch 15, **dadurch gekennzeichnet, dass** der Sensor (4) einen piezo-elektrischen Torsionsschwingerkristall (8) aufweist.

**18.** Vorrichtung nach Anspruch 15, **dadurch gekennzeichnet, dass** der Sensor (4) einen Temperatur-Detektor und eine Einrichtung zur Bestimmung der relativen Permittivität aufweist.

**19.** Vorrichtung nach Anspruch 15, **dadurch gekennzeichnet, dass** der Sensor (4) in der Ölwanne (3) des Verbrennungsmotors (1) angeordnet ist.

**20.** Vorrichtung nach Anspruch 15, **dadurch gekennzeichnet, dass** ein Bedienelement (7) zum Auslösen eines Messvorgangs nach einem Wechsel des Schmieröls vorgesehen ist.
